Europäisches Patentamt

**(19)** European Patent Office

Office européen des brevets

**(11)** Publication number: **0 295 116**
**A1**

**(12)** EUROPEAN PATENT APPLICATION

**(21)** Application number: **88305305.0**

**(22)** Date of filing: **10.06.88**

**(51)** Int. Cl.⁴: $A 61 K 7/16$

**(30)** Priority: **12.06.87 GB 8713748**
**21.10.87 GB 8724654**

**(43)** Date of publication of application:
**14.12.88 Bulletin 88/50**

**(84)** Designated Contracting States:
**AT BE CH DE ES FR GB GR IT LI NL SE**

**(71)** Applicant: **UNILEVER PLC**
**Unilever House Blackfriars P.O. Box 68**
**London EC4P 4BQ (GB)**

**(84)** Designated Contracting States: **GB**

**(71)** Applicant: **UNILEVER NV**
**Burgemeester s'Jacobplein 1 P.O. Box 760**
**NL-3000 DK Rotterdam (NL)**

**(84)** Designated Contracting States:
**BE CH DE ES FR GR IT LI NL SE AT**

**(72)** Inventor: **Ingram, Geoffrey Stewart The Cairngorms**
**4 Stanley Avenue Bebington**
**Wirral Merseyside L63 5QF (GB)**

**(74)** Representative: **Doucy, Robert Henry et al**
**Unilever PLC Patents Division P.O. Box 68 Unilever**
**House**
**London EC4P 4BQ (GB)**

**(54)** Oral compositions.

**(57)** The disclosure relates to an oral composition for inhibiting the formation of dental calculus comprising in combination a polyphosphate salt and a zinc salt.

EP 0 295 116 A1

Bundesdruckerei Berlin

**Description**

## ORAL COMPOSITIONS

This invention relates to oral compositions containing an anti-calculus agent.

There have recently been published proposals to formulate anti-calculus oral products on the basis of linear molecularly dehydrated polyphosphate salts. Such products are disclosed in US-A-4 627 977 (Gaffar et al), US-A-4 515,772 (Parran et al), US-A-4 590 066 (Parran et al), US-A-4 684 518 (Parran et al), GB-A-2 182 244 (Colgate-Palmolive) and GB-A-2 188 548 (Beecham), all incorporated herein by reference. Examples of polyphosphate salts that may be present in such compositions as given in these patents are hexametaphosphates, tripolyphosphates and pyrophosphates. Prior to the publication of the above-mentioned patents it had been disclosed in US-A-4 100 269 (Pader), incorporated herein by reference, that certain zinc salts, for example zinc citrate, exhibited an anti-calculus effect when incorporated in dentifrices.

US-A-4 627 977 (Gaffar et al) warns against the inclusion of zinc salts in oral products containing the polyphosphate salts because of complex formation and states that the presence of zinc salts should be avoided.

Contrary to the teaching of US-A-4 627 977 (Gaffar et al) we have found that the combination of a zinc salt, such as zinc citrate, and a polyphosphate salt, such as sodium tripolyphosphate or potassium pyrophosphate, is advantageous providing an effective stable anti-caries composition.

According to the present invention there is provided an oral composition, preferably a toothpaste, comprising the combination of a zinc salt and a linear molecularly dehydrated polyphosphate salt. The polyphosphate salt may be any of those described in US-A-4 627 977 (Gaffar et al), US-A-4 515 772 (Parran et al) or GB-A-2 182 244 (Colgate-Palmolive) and may be used in amounts as also disclosed in those patents. Similarly the zinc salt may be any of the sparingly soluble salts disclosed in US-A-4 100 269 (Pader). More water-soluble salts may also be used as disclosed in US-A-4 022 880, incorporated herein by reference. Other zinc salts suitable for use in oral compositions are disclosed in US-A-4 144 323 (Lamberti), US-A-4 656 031 (Lane et al) and US-A-4 160 821 (Sipos), all incorporated herein by reference.

A preferred linear molecularly dehydrated polyphosphate salt is sodium tripolyphosphate. Other preferred salts are the alkali metal pyrophosphates, for example di- and tetra-sodium pyrophosphate and di- and tetra-potassium pyrophosphates and mixtures thereof. The pyrophosphates are preferably used in an amount sufficient to provide at least 0.5%, more preferably at least 1.5%, $P_2O_7^{4-}$ ion.

One embodiment of an anti-calculus oral composition according the invention comprises 0.5 to 5%, preferably 1% to 3%, by weight sodium tripolyphosphate and 0.05 to 2%, preferably 0.1 to 1%, by weight zinc citrate.

A further particular embodiment of an anti-calculus composition according to the invention comprises 0.5 to 7%, preferably 1% to 5%, by weight of a mixture of the tetra alkali metal salts such as a mixture of sodium and potassium pyrophosphates, and 0.05 to 2%, preferably 0.1 to 1%, by weight of a zinc salt such as zinc citrate.

The oral composition of the invention may also include a source of fluoride-ions or fluorine providing compound. An amount of a fluoride source sufficient to supply from about 50ppm to about 3500ppm of fluoride ions may be used. Preferred fluorides are sodium fluoride and sodium monofluorophosphate. Other suitable sources of fluoride ions are disclosed in the patents referred to above.

If considered desirable the fluoride-ion source may be present as part of a combination inhibitor against enzymatic hydrolysis of the polyphosphate salt where the other component of the inhibitor is a synthetic anionic polymeric polycarboxylate as described in GB-A-2 182 244 (Colgate-Palmolive).

If desired other anti-calculus agents may also be present such as the acrylic polymer or copolymers described in US-A-4 661 341 (Benedict et al) or the polycarboxylic polymers proposed in US-A-3 429 963 (Shedlovsky), both incorporated herein by reference.

Where the oral composition of the invention is a toothpaste, the composition may also comprise an abrasive cleaning agent. While several abrasive agents suitable for toothpaste use are described in the above-mentioned prior patents, preferred abrasives are silicas, water-insoluble sodium metaphosphate and plastics materials, or mixtures thereof.

Other ingredients of oral compositions are well known to those skilled in the art. In any event, they are generally described in the aforementioned patents. Thus toothpastes will generally also comprise a humectant, binder or thickener, and surfactant.

The oral compositions of the invention may be made by standard procedures which involve blending the polyphosphate salt and zinc salt with other conventional ingredients.

The following Examples illustrate toothpaste compositions in accordance with the invention which are effective to reduce the growth of calculus. Percentages are by weight.

Example 1
A toothpaste is prepared having the following composition.

%

| | % |
|---|---|
| Silica xerogel | 12.0 |
| Sorbitol syrup (70% solution) | 35.0 |
| Glycerol | 26.0 |
| Hydroxyethyl cellulose | 1.7 |
| Sodium lauryl sulphate | 1.5 |
| Zinc citrate trihydrate | 0.5 |
| Sodium tripolyphosphate | 1.0 |
| Sodium fluoride | 0.23 |
| Saccharin | 0.20 |
| Flavour | 1.00 |
| Water | to 100.00 |

Examples 2 to 6

Other examples of formulation are made by varying the amount of zinc citrate and/or sodium tripolyphosphate as indicated below:

| | zinc citrate(%) | sodium tripolyphosphate(%) |
|---|---|---|
| Example 2 | 1.0 | 1.0 |
| Example 3 | 0.5 | 2.5 |
| Example 4 | 1.0 | 2.5 |
| Example 5 | 0.5 | 5.0 |
| Example 6 | 1.0 | 5.0 |

Example 7

A toothpaste is prepared having the following composition.

%

| | |
|---|---|
| Silica xerogel | 12.00 |
| Precipitated silica | 8.00 |
| Sorbitol syrup (70% solution) | 40.00 |
| Xanthan gum | 1.00 |
| Sodium lauryl sulphate | 1.50 |
| Zinc citrate trihydrate | 0.50 |
| Tetrasodium pyrophosphate decahydrate | 2.56 |
| Sodium fluoride | 0.22 |
| Titanium dioxide | 0.50 |
| Saccharin | 0.20 |
| Flavour | 1.00 |
| Water | to 100.00 |

Example 8
A toothpaste is prepared having the following composition.

%

| | |
|---|---|
| Silica abrasive agent | 10.00 |
| Silica thickening agent | 8.00 |
| Sorbitol syrup (70% solution) | 40.00 |
| Xanthan gum | 0.60 |
| Polyethylene glycol (1500) | 5.00 |
| Sodium lauryl sulphate | 1.70 |
| Dodecyl benzene sulphonate | 0.50 |
| Zinc citrate trihydrate | 0.50 |
| Tetrapotassium pyrophosphate | 0.62 |
| Tetrasodium pyrophosphate | 0.50 |
| Sodium fluoride | 0.24 |
| Titanium dioxide | 1.00 |
| Saccharin | 0.25 |
| Flavour | 1.20 |
| Formalin | 0.04 |
| Water | to 100.00 |

Example 9

A toothpaste is made having the composition of Example 8 except that the amount of tetrapotassium pyrophosphate is 1.86% and the amount of tetrasodium pyrophosphate is 1.5%.

**Claims**

1. An oral composition for inhibiting the formation of dental calculus comprising 0.5 to 7% by weight of a linear molecularly dehydrated polyphosphate salt and 0.05 to 2% by weight of a zinc salt.

2. An oral composition as claimed in claim .1 wherein the polyphosphate salt is sodium tripolyphosphate.

3. An oral composition as claimed in claim 1 wherein the polyphosphate salt is a pyrophosphate.

4. An oral composition as claimed in claim 3 wherein the pyrophosphate is a di- or tetra-alkali metal pyrophosphate or a mixture thereof.

5. An oral composition as claimed in claim 4 wherein the pyrophosphate is a mixture of tetrasodium and tetrapotassium pyrophosphates.

6. An oral composition as claimed in any of claims 3 to 5 wherein the pyrophosphate is present in an amount sufficient to provide at least 0.5% $P_2O_7^{4-}$.

7. An oral composition as claimed in claim 6 wherein the pyrophosphate is present in an amount sufficient to provide at least 1.5% $P_2O_7^{4-}$.

8. An oral composition as claimed in any of the preceding claims comprising 0.1 to 1% by weight of zinc salt.

9. An oral composition as claimed in any of the preceding claims wherein the zinc salt is zinc citrate.

10. An oral composition as claimed in claim 1 comprising 1 to 7% by weight of a pyrophosphate selected from di- and tetra- sodium and potassium pyrophosphates and 0.1 to 2% by weight of a zinc salt.

11. An oral composition as claimed in any of the preceding claims in the form of a toothpaste.

12. Method of making an oral composition as claimed in any of the preceding claims comprising blending the respective specified ingredients with other conventional ingredients.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 989 814  (M. CORDON et al.)<br>* column 6, example; claims * | 1-12 | A 61 K    7/16 |
| A | GB-A-1 290 627  (UNILEVER LTD.)<br>* page 4, table 2; claims * | 1-12 | |
| A | EP-A-0 097 476  (THE PROCTER & GAMBLE CO.)<br>* examples I,II; claims * & US - A - 4 515 772 (Cat. D) | 1-12 | |
| A | US-A-4 627 977  (A. GAFFAR et al.)<br>* claim 1 * | 1-12 | |
| A | EP-A-0 161 898  (UNILEVER PLC)<br>* page 26; claim 8 * & US - A - 4 656 031 (Cat. D) | 1-12 | |

TECHNICAL FIELDS
SEARCHED (Int. Cl.4)

A 61 K    7/16

The present search report has been drawn up for all claims

| Place of search | Date of completion of the search | Examiner |
|---|---|---|
| BERLIN | 05-08-1988 | AVEDIKIAN P.F. |